# FASCICULE DE BREVET EUROPEEN

(11) **EP 1 003 710 B1**
(45) Date de publication et mention de la délivrance du brevet: **13.02.2002**
(21) Numéro de dépôt: 98942728.1
(22) Date de dépôt: 11.08.1998
(51) Int. Cl.: C07C 201/16, C07C 205/06

(54) **PROCEDE DE TRAITEMENT DE SOLUTIONS AQUEUSES COMPRENANT DES ACIDES ET DES COMPOSES NITRES**
VERFAHREN ZUR AUFARBEITUNG VON SÄURE UND NITROVERBINDUNGEN ENTHALTENDEN WÄSSRIGEN LÖSUNGEN
METHOD FOR TREATING AQUEOUS SOLUTIONS COMPRISING ACIDS AND NITRATED COMPOUNDS

(30) Priorité: 13.08.1997 FR 9710346
(43) Date de publication de la demande: 31.05.2000
(73) Titulaire: RHODIA CHIMIE, 92512 Boulogne Billancourt Cedex (FR)
(72) Inventeur: MARION, Philippe, F-69390 Vernaison (FR); LE BRIS, Louis, F-69005 Lyon (FR); BERROD, Gérard, F-69100 Villeurbanne (FR); DOVERGNE, Georges, F-38800 Le Pont de Claix (FR); PERRONA, Philippe, F-69390 Charly (FR)
(74) Mandataire: Wattremez, Catherine
(86) Numéro de dépôt international: FR9801793
(87) Numéro de publication internationale: WO9908995

(56) Documents cités:
- EP-A- 0 047 331
- US-A- 2 773 911

## Description

Le procédé selon l'invention concerne le traitement de solutions aqueuses issues de lavages, et plus particulièrement de lavages acides de composés aromatiques nitrés.

Les procédés de préparation de composés nitrés aromatiques, et plus particulièrement de composés aromatiques dinitrés, sont exploités industriellement depuis de nombreuses années. Ces composés sont en effet des intermédiaires pour la préparation de diamines aromatiques, ces dernières étant utilisées pour la synthèse des isocyanates correspondants. Les isocyanates sont pour leur part employés pour la synthèse de polyuréthannes, dont les applications sont très nombreuses.

Résumées rapidement, les réactions de dinitration sont mises en oeuvre, la plupart du temps, en deux étapes, la première consistant à préparer les composés mononitrés, la seconde, les composés dinitrés. Outre le composé aromatique à faire réagir, on emploie un acide nitrant, qui est en général un mélange acide nitrique / acide sulfurique, l'acide sulfurique étant un catalyseur de la réaction.

A l'issue de chaque étape de nitration, le composé nitré est séparé de l'acide résiduaire. Cette opération a lieu classiquement par décantation directe ou par centrifugation du mélange réactionnel.

Les composés nitrés obtenus après séparation d'avec l'acide nitrant (aussi appelés composés nitrés bruts) ne peuvent être utilisés tels quels, sauf s'ils sont destinés à être à nouveau nitrés. En effet, ils contiennent toujours, à l'état dissous, une fraction d'acide nitrant, de même que des impuretés organiques.

Il est fait remarquer que les réactions de nitration mises en oeuvre au moyen d'acide nitrique seul comme acide nitrant, font apparaître les mêmes difficultés de purification des composés nitrés obtenus.

En effet, les composés aromatiques nitrés ainsi séparés de l'acide résiduaire, contiennent eux aussi, une fraction d'acide nitrique, représentant quelques pourcents en poids des composés nitrés, qu'il est nécessaire de récupérer. Ils contiennent de même des sous-produits organiques qu'il faut éliminer.

Ces problèmes liés à la purification des composés nitrés aromatiques, et plus particulièrement à la séparation et à la récupération des acides dissous, ont fait l'objet de nombreuses études.

En effet, récupérer ces acides résiduaires représente un enjeu économique non négligeable, étant donnés les tonnages de composés nitrés. Par ailleurs, leur récupération a aussi un impact sur l'environnement car elle a notamment pour conséquence de limiter les rejets aqueux. De plus, la récupération des acides permet de diminuer les coûts nécessaires pour traiter les eaux résiduaires que l'on ne peut rejeter telles quelles, car elles sont polluées par des sels, tels que les sulfates et surtout les nitrates.

Les méthodes de séparation et de récupération des acides nitrique et sulfurique dissous ont toutes trait à l'augmentation de l'efficacité de divers lavages des composés nitrés.

Par exemple, dans le brevet US 2 773 911, ayant pour objet un procédé de préparation de mononitrobenzène (MNB) en continu, il est proposé de récupérer l'acide sulfurique. Ainsi, lorsque la réaction de nitration est terminée, le mélange réactionnel est décanté pour donner une phase aqueuse comprenant les acides (1) n'ayant pas réagi, et une phase organique comprenant le MNB (2). La phase organique (2) est lavée en présence d'eau. Le MNB récupéré est pur et peut être stocké ou utilisé pour une réaction ultérieure. La phase aqueuse (3) ainsi obtenue est ensuite mise en contact avec du benzène pour en extraire le MNB entraîné. La phase aqueuse résultante (4) est purgée, de préférence à un bassin de neutralisation. La phase organique (5) récupérée, est mise en contact avec la phase aqueuse (1) mentionnée auparavant, dans le but d'extraire le MNB entraîné, et de faire réagir l'acide nitrique résiduel avec le benzène. La phase aqueuse (6), comprenant de l'acide sulfurique est concentrée pour être réutilisée, tandis que la phase organique (7) est recyclée dans la réaction de nitration.

Ce procédé ne propose toutefois pas de récupérer et traiter la phase aqueuse (4).

Selon une autre possibilité, décrite notamment dans le brevet européen EP 279 312, on effectue le lavage du dinitrotoluène brut avec une quantité d'eau très faible. De cette façon, les eaux de lavages, chargées en acides nitrique et sulfurique, sont suffisamment concentrées pour pouvoir être renvoyées directement dans le processus de nitration. Cependant, un tel procédé présente l'inconvénient de nécessiter l'emploi d'un appareillage particulier pour séparer la phase aqueuse de la phase organique, en l'occurrence, un coalesceur. En effet, du fait de la très faible teneur en eau utilisée pour le lavage, la décantation est difficile. En outre, les rendements d'extraction sont d'au plus 72 %.

Une autre possibilité, ayant fait l'objet de la demande de brevet européen EP 736 514, consiste à laver en plusieurs étapes à contre-courant le dinitrotoluène brut avec de l'eau chargée en acides employés pour la réaction de nitration. Les eaux récupérées peuvent être recyclées dans le procédé de nitration, sans concentration préalable, ou de préférence avec une concentration préalable des eaux acides. Ce procédé ne présente cependant, pas toutes les garanties de sécurité souhaitées. En effet, l'étape de concentration des eaux acides, qui s'avère dans la majeure partie des cas nécessaire, présente quelques risques, car la phase aqueuse à distiller comprend à la fois les acides nitrants et du dinitrotoluène dissous. Par conséquent, on se trouve dans des conditions de nitration d'un dinitrotoluène, qui conduit au trinitrotoluène dont on connaît les propriétés particulières. De plus, les dinitrotoluènes présents dans la phase aqueuse acide issue du lavage ne sont pas récupérés, dans les cas où la concentration desdites eaux est mise en oeuvre. En effet, ces composés sont entraînés avec l'eau lors de la distillation où ils sont perdus. Par ailleurs, les composés nitrés peuvent être la cause d'un bouchage ou d'un encrassement de la colonne de concentration car ils sont condensés en tête de colonne et se retrouvent alors sous une forme solide. Ils peuvent de même se retrouver dans les eaux destinées à être rejetées, causant alors une pollution ou un surcoût car il est nécessaire de les éliminer.

Ainsi, la présente invention a pour objet de récupérer le ou les acides contenus dans les composés nitrés bruts, en mettant en oeuvre un procédé simple, efficace et présentant le maximum de garanties sur le plan de la sécurité lors de la mise en oeuvre.

En outre, le procédé selon l'invention permet de récupérer de même les composés nitrés aromatiques qui seraient dissous dans la phase aqueuse issue du lavage des composés nitrés bruts.

Ainsi, ces buts et d'autres sont atteints par l'invention qui a pour objet le traitement d'une solution aqueuse issue du lavage de composés aromatiques mononitrés ou dinitrés obtenus par la réaction d'un composé aromatique correspondant, avec un acide nitrant comprenant au moins de l'acide nitrique, lesdits composés aromatiques mononitrés ou dinitrés ayant été, préalablement au lavage mentionné, séparés de la phase aqueuse acide ; ledit lavage étant effectué avec de l'eau pouvant aussi comprendre des traces d'acide nitrant, caractérisé en ce que l'on met en oeuvre les étapes suivantes :
(a) on met en contact ladite solution aqueuse avec le composé aromatique précité, et l'on obtient une phase aqueuse et une phase organique,
(b) on recycle ladite phase organique dans le procédé de nitration, ou dans l'extraction des acides résiduaires de mononitration,
(c) on distille ladite phase aqueuse,
(d) on recycle la solution acide concentrée ainsi obtenue, dans le procédé de nitration,
(e) on recycle ou on élimine l'eau récupérée après la distillation.

Mais d'autres caractéristiques et avantages de la présente invention apparaîtront plus clairement à la lecture de la description qui va suivre.

Ainsi que cela a été indiqué auparavant, le procédé selon l'invention est tout particulièrement approprié pour traiter les solutions aqueuses issues de procédés de nitration de composés aromatiques.

Les composés aromatiques susceptibles d'être nitrés peuvent comprendre un ou plusieurs noyaux aromatiques. Conviennent par exemple le benzène et ses dérivés, le naphtalène et ses dérivés, le phénanthrène et ses dérivés, le biphényle, l'oxyde de diphényle et leurs dérivés.

Par dérivés, on désigne des radicaux aromatiques comportant un ou plusieurs substituants, tels que les radicaux alkyle C₁-C₆ ou cycloalkyle en C₃-C₆ ; des radicaux hydroxyle ; des radicaux alcoxy en C₁-C₅ ; des radicaux aminoacylés en C₁-C₄ ; des atomes d'halogène.

A titre d'exemple de radical alkyle ou cycloalkyle, on peut citer, sans intention de s'y limiter, le méthyle, l'éthyle, le n-propyle, l'isopropyle, le n-butyle, l'isobutyle, le tertiobutyle, le n-hexyle, le cyclohexyle.

En ce qui concerne les radicaux alcoxy, on peut citer par exemple, le méthoxy, l'éthoxy, le propoxy.

En tant que radicaux aminoacylés, conviennent notamment les radicaux acétylamine, benzoylamine.

Tous les halogènes conviennent, que ce soit le fluor, le chlore, le brome et l'iode.

De préférence, le composé aromatique mis en oeuvre dans les réactions de nitration, est choisi parmi le benzène, le toluène, le xylène et ses isomères, l'éthylbenzène, le propylbenzène, l'isopropylbenzène, le chlorobenzène, le chlorométhylbenzène, le chloréthylbenzène, le biphényle, l'oxyde de diphényle.

Selon un mode de réalisation particulier, le flux traité conformément à la présente invention, provient de la nitration du benzène, soit le mononitrobenzène ou les différents isomères du dinitrobenzène. Le procédé selon l'invention est de même particulièrement approprié pour traiter les solutions aqueuses issues du lavage de composés obtenus par nitration du toluène, soit les différents isomères du mononitrotoluène, et préférentiellement, ceux du dinitrotoluène.

Il est à noter que la présente invention conviendrait de même pour le traitement de solutions aqueuses issues du lavage des composés aromatiques précités, ayant fait l'objet d'une trinitration.

Avant de définir plus en détails le procédé de l'invention proprement dit, il est nécessaire de décrire la façon dont sont obtenus les solutions aqueuses à traiter conformément à l'invention.

Tout d'abord, le procédé selon l'invention convient parfaitement au traitement de composés nitrés obtenus en mettant en oeuvre l'acide nitrique seul, ou un mélange sulfonitrique, comme acide nitrant.

Dans le cas où l'acide nitrique seul est mis en oeuvre, la séparation des composés nitrés de l'acide restant est mise en oeuvre en deux temps. On introduit tout d'abord dans le mélange réactionnel un sel choisi parmi les nitrates, puis on vaporise partiellement le mélange ainsi additivé. Il devient alors possible de séparer par décantation les composés nitrés de la phase aqueuse qui comprend l'acide nitrique et le nitrate.

Dans le cas où la nitration est effectuée en présence d'un mélange sulfonitrique, la séparation est immédiate en utilisant des moyens connus comme une simple décantation ou une centrifugation.

Dans ce qui va suivre, il sera fait référence à des composés nitrés obtenus en mettant en oeuvre un mélange sulfonitrique comme acide nitrant. Bien entendu, tout ce qui est indiqué pour un mélange nitrant reste valable si seul l'acide nitrique est employé pour la réaction de nitration.

Les composés nitrés obtenus, qualifiés de composés nitrés bruts, sont donc mis en contact avec un milieu lavant comprenant de l'eau (lavage dit acide). Cette opération a pour but de séparer desdits composés bruts, les faibles quantités d'acide nitrant solubles ou entraînées lors de la décantation.

Bien souvent, le lavage acide comprend plusieurs étapes, de manière à augmenter l'efficacité de l'extraction.

Notons de plus que le milieu lavant mis en oeuvre lors d'une étape du lavage acide, est à base d'eau, mais il peut aussi comprendre des traces d'acide nitrant. En effet, ces traces d'acides nitrant peuvent avoir été extraites, par exemple lors d'étapes antérieures de ce même lavage acide (utilisation du milieu lavant de l'étape n-1 pour l'étape n), ou encore peuvent provenir d'un recyclage dans l'étape en question, d'une fraction du milieu lavant issu de cette même étape du lavage acide (bloucle).

Selon un premier mode de réalisation, l'opération de lavage a lieu dans une colonne d'extraction. Selon cette possibilité, la colonne comprend de 1 à 6 étages théoriques. Dans ce cas, on met en oeuvre un lavage à contre-courant, l'alimentation en composés nitrés bruts et en milieu lavant ayant lieu à des extrémités opposées de la colonne.

Selon un second mode de réalisation, le lavage a lieu dans au moins une unité mélangeur - décanteur. Dans le cas où plusieurs unités de ce type sont employées, il est préférable d'effectuer le lavage à contre-courant, de manière à améliorer l'efficacité de l'extraction.

Afin de limiter la quantité de milieu lavant tout en conservant de bonnes conditions d'extraction, il est préférable, pour chaque unité mélangeur - décanteur, de recycler une partie du milieu lavant dans la même unité, l'autre étant alimentée à une unité, de préférence en amont (fonctionnement à contre-courant).

En outre, pour conserver le fonctionnement en mode continu, il est classique de faire un appoint en milieu lavant, correspondant à la quantité de solution aqueuse soutirée qui va faire l'objet du traitement selon l'invention. Notons que le milieu lavant peut être de l'eau propre ou bien encore de l'eau provenant de tout autre point du procédé, comme par exemple, d'une étape de lavage neutre des composés nitrés aromatiques.

Le milieu lavant comprend par conséquent au moins de l'eau. Selon un mode de mise en oeuvre plus particulier, le milieu lavant comprend à la fois de l'eau et de l'acide nitrant. On se trouve dans une telle situation si le lavage a lieu à contre-courant, comme c'est le cas pour les colonnes d'extraction, ou bien encore pour l'emploi de plusieurs unités mélangeurs - décanteurs en série.

Selon l'un et l'autre des modes de réalisation précités, la teneur globale en milieu lavant par rapport aux composés nitrés à laver est telle que le rapport massique de la phase comprenant les composés nitrés par rapport à l'eau est compris entre 1,5 et 6.

Quelque soit la variante retenue de lavage, ce dernier doit être mis en oeuvre à une température supérieure à la température de fusion des composés nitrés à laver.

Une fois le lavage effectué, on récupère des composés nitrés qui peuvent subir d'autres étapes de lavages.

Classiquement, on met en oeuvre un lavage qualifié de basique. Le but d'une telle opération est de séparer des composés nitrés, les sous-produits organiques, qui sont pour la majeure partie des composés hydroxynitroaromatiques, en les transformant en sels qui sont alors solubles dans la phase aqueuse. La base employée peut être choisie parmi les hydroxydes de métaux alcalins ou encore les carbonates de métaux alcalins. On pourra se référer à la demande de brevet européen EP 662 454 dans laquelle est décrit un tel lavage basique.

Il est de même possible, à l'issue de ce lavage basique d'effectuer un dernier lavage à l'eau (dit lavage neutre) afin d'éliminer toute trace de la base utilisée lors du lavage précédent. Les composés nitrés résultants peuvent alors être hydrogénés selon les méthodes classiques.

Le traitement selon l'invention de la solution aqueuse issue de la première catégorie de lavage, dit lavage acide, va maintenant être décrit.

La solution aqueuse issue de ce lavage acide, comprend outre l'eau, des composés nitrés aromatiques, et l'acide nitrant.

Dans une première étape (a), ladite solution aqueuse est mise en contact avec un composé aromatique. Ce composé aromatique est plus particulièrement celui qui fait l'objet du procédé de nitration en amont. En d'autres termes, le composé aromatique utilisé pour l'extraction est celui mis en oeuvre pour la réaction de mononitration.

La mise en contact peut avoir lieu dans une colonne d'extraction ou bien dans au moins une unité mélangeur - décanteur.

Dans le cas où une colonne est mise en oeuvre, le nombre de plateaux théoriques est plus particulièrement compris entre 2 et 10.

L'alimentation du composé aromatique et celle de la solution aqueuse ont lieu à chacune des extrémités de la colonne, la solution aqueuse étant de préférence introduite en tête de la colonne, le composé aromatique, en pied. Classiquement, les alimentations ont lieu au moins un plateau au-dessus du premier et au moins un plateau au-dessous du dernier.

Dans le cas où l'option mélangeur - décanteur est retenue, il est préférable de la mettre en oeuvre avec au moins deux unités mélangeur - décanteur, et de préférence dans une série comprenant de 2 à 10 unités.

Encore plus particulièrement selon cette option, on recycle une partie du flux de composé aromatique dans la même unité mélangeur - décanteur, l'autre partie étant alimentée dans une unité mélangeur - décanteur située en amont ou en aval, selon que le mode de fonctionnement choisi est respectivement l'extraction à contre-courant ou non. Dans ce cas, et pour maintenir un fonctionnement en continu, on fait un appoint en composé aromatique propre. De préférence, cet appoint est réalisé au dernier étage de lavage, ou bien encore à l'opposé de l'alimentation en phase aqueuse dans la colonne-fonctionnement à contre-courant.

Selon un mode de réalisation particulier de cette option, on met en oeuvre l'extraction à contre-courant.

La quantité de composé aromatique mise en oeuvre par rapport à celle de la solution aqueuse à traiter varie dans un large domaine. A titre illustratif, le rapport pondéral de la solution aqueuse au composé aromatique est compris entre 1/1 et 10/1.

Habituellement, cette opération est conduite à une température de l'ordre de 10 à 70°C, de préférence à une température comprise entre 40 et 60°C.

On récupère à l'issue de cette étape (a) une phase organique comprenant majoritairement le composé aromatique, employé comme solvant d'extraction, dans lequel est dissoute la fraction de composés aromatiques nitrés qui se trouvait à l'origine dans la solution aqueuse.

De manière tout à fait avantageuse, ladite phase organique, dans une étape (b) est recyclée dans le processus de nitration. Selon un mode de réalisation préféré, que l'on mette en oeuvre un procédé de mono ou de dinitration, ladite phase organique est réintroduite à l'étape de mononitration. Par exemple, ladite phase organique peut être engagée dans la réaction de mononitration, ou bien encore employée pour l'extraction des acides résiduaires de mononitration, c'est-à-dire de la phase aqueuse acide obtenue après décantation du mélange réactionnel issu de la réaction de mononitration.

Quant à la phase aqueuse issue de l'étape (a) c'est-à-dire obtenue après la mise en contact de la solution aqueuse issue du lavage des composés nitrés bruts avec le composé aromatique, elle est dans une étape (c) distillée. Cette opération a pour objet de concentrer ces eaux qui comprennent des acides.

Il est à noter que cette opération de concentration peut avoir lieu sans aucun danger car les composés aromatiques nitrés ont préalablement été extraits.

Cette opération de concentration a lieu bien entendu, et ce de manière classique, dans au moins une colonne de distillation. Cette colonne comprend un nombre de plateaux théoriques compris plus particulièrement entre 3 et 10.

Afin de minimiser les coûts énergétiques, on peut envisager d'effectuer cette concentration au moyen de deux colonnes. Dans une première colonne, on effectue la vaporisation d'une partie de l'eau. Dans la seconde, la vaporisation de l'eau est conduite jusqu'à obtenir la concentration souhaitée en acides. Cette colonne est alimentée par la fraction liquide récupérée en pied de la colonne précédente, et chauffée par le flux thermique récupéré lors de la condensation de l'eau en tête de la première colonne.

Selon ce cas de figure, la première colonne comprend plus avantageusement un étage théorique, voire éventuellement deux. La seconde serait une colonne présentant au moins trois étages théoriques.

La ou les colonnes fonctionnent de manière avantageuse sous pression atmosphérique mais un fonctionnement sous vide, de l'une et/ou l'autre des colonnes, est parfaitement envisageable.

Le procédé selon l'invention permet d'obtenir des solutions d'acides dont la concentration totale en acides est comprise entre 30 et 75 %.

La solution concentrée comprenant l'acide est avantageusement recyclée dans le procédé de nitration proprement dit (étape (d)), que ce soit à l'étape de mononitration, ou à celle de dinitration.

Les eaux effluentes récupérées en tête de colonne, à l'issue de l'étape (c) peuvent être rejetées telles quelles, mais elles peuvent aussi être recyclées dans le lavage des composés nitrés aromatiques bruts (étape (e)), qu'ils soient mononitrés ou dinitrés.

## Revendications

1. Procédé de traitement d'une solution aqueuse issue du lavage de composés aromatiques mononitrés ou dinitrés obtenus par la réaction d'un composé aromatique correspondant, avec un acide nitrant comprenant au moins de l'acide nitrique, lesdits composés aromatiques mononitrés ou dinitrés ayant été, préalablement au lavage mentionné, séparés de la phase aqueuse acide : ledit lavage étant effectué avec de l'eau pouvant aussi comprendre des traces d'acide nitrant, **caractérisé en ce que** l'on met en oeuvre les étapes suivantes :
(a) on met en contact ladite solution aqueuse avec le composé aromatique précité, et l'on obtient une phase aqueuse et une phase organique.
(b) on recycle ladite phase organique dans le procédé de nitration, ou dans l'extraction des acides résiduaires de mononitration,
(c) on distille ladite phase aqueuse.
(d) on recycle la solution acide concentrée ainsi obtenue, dans le procédé de nitration,
(e) on recycle ou on élimine l'eau récupérée après la distillation.

2. Procédé selon la revendication précédente **caractérisé en ce que** l'on effectue l'étape (a) dans une colonne d'extraction, ou dans au moins une unité mélangeur-décanteur, de préférence au moins deux unités mélangeur - décanteur.

3. Procédé selon la revendication précédente, **caractérisé en ce que** l'étape (a) est mise en oeuvre dans une colonne d'extraction dans laquelle la solution aqueuse est alimentée en tête de la colonne et le composé aromatique en pied de la colonne.

4. Procédé selon la revendication 2, **caractérisé en ce que** l'on recycle une partie du flux de composé aromatique dans la même unité mélangeur - décanteur, l'autre partie étant alimentée dans une unité mélangeur - décanteur située en amont ou en aval, selon que le mode de fonctionnement choisi est respectivement l'extraction à contre-courant ou non.

5. Procédé selon la revendication précédente, **caractérise en ce que** l'étape (a) est mise en oeuvre dans au moins deux unités mélangeur décanteur, fonctionnant à contre-courant.

6. Procédé selon l'une quelconque des revendications précédentes, **caractérisé en ce que** l'on effectue la mise en contact de l'étape (a) à une température comprise entre 10 et 70°C, de préférence entre 40 et 70 °C.

7. Procédé selon l'une quelconque des revendications précédentes, **caractérisé en ce que** l'on effectue la mise en contact de l'étape (a) avec un rapport pondéral de la solution aqueuse au composé aromatique compris entre 1/1 et 10/1.

8. Procédé selon l'une quelconque des revendications précédentes, **caractérisé en ce que** l'on effectue la distillation de l'étape (c) dans au moins une colonne.

9. Procédé selon l'une quelconque des revendications précédentes, **caractérisé en ce que** l'on effectue la distillation pour obtenir une solution d'acides dont la concentration totale en acides est comprise entre 30 et 75 %.

10. Procédé selon l'une quelconque des revendications précédentes, **caractérisé en ce que** l'on effectue la distillation de l'étape (c) dans deux colonnes :
la première colonne dans laquelle une partie de l'eau est vaporisée.
la seconde colonne dans laquelle la vaporisation est conduite jusqu'à obtenir la concentration souhaitée en acides, étant alimentée par la fraction liquide récupérée en pied de la colonne précédente, et chauffée par le flux thermique récupéré lors de la condensation de l'eau en tête de la première colonne.

11. Procédé selon l'une quelconque des revendications précédentes, **caractérisé en ce que** l'on traite des solutions aqueuses issues du lavage de composés aromatiques choisis parmi le mononitrobenzène, le dinitrobenzène et ses isomères, le mononitrotoluène, le dinitrotoluène et ses isomères.

12. Procédé selon l'une quelconque des revendications précédentes, **caractérisé en ce que** l'on met en oeuvre l'étape (a) avec un composé aromatique choisi parmi le benzène, le toluène.

## Patentansprüche

1. Verfahren zur Behandlung einer wäßrigen Lösung, die aus der Wäsche von mononitrierten oder dinitrierten aromatischen Verbindungen, die durch Reaktion einer entsprechenden aromatischen Verbindung erhalten worden sind, mit einer Nitriersäure stammt, die mindestens Salpetersäure enthält, wobei die mononitrierten oder dinitrierten aromatischen Verbindungen vor der erwähnten Wäsche von der sauren wäßrigen Phase abgetrennt worden sind, wobei die Wäsche mit Wasser durchgeführt wird, das auch Spuren an Nitriersäure enthalten kann, **dadurch gekennzeichnet, daß** man die folgenden Verfahrensschritte durchführt:
(a) Man bringt die wäßrige Lösung mit der zuvor genannten aromatischen Verbindung in Kontakt und erhält eine wäßrige Phase und eine organische Phase.
(b) Man wiederverwendet die organische Phase im Nitrierprozeß oder bei der Extraktion der restlichen Säuren aus der Mononitrierung.
(c) Man destilliert die wäßrige Phase.
(d) Man wiederverwendet die so erhaltene, konzentrierte Säurelösung in dem Nitrierprozeß.
(e) Man wiederverwendet oder entfernt das nach der Destillation erhaltene Wasser.

2. Verfahren nach dem vorhergehenden Anspruch, **dadurch gekennzeichnet, daß** man Verfahrensschritt (a) in einer Extraktionskolonne oder in mindestens einer Einheit Mischer-Dekanter, vorzugsweise mindestens zwei Einheiten Mischer-Dekanter, durchführt.

3. Verfahren nach dem vorhergehenden Anspruch, **dadurch gekennzeichnet, daß** Verfahrensschritt (a) in einer Extraktionskolonne durchgeführt wird, in welcher die wäßrige Lösung am Kopf der Kolonne und die aromatische Verbindung am Fuß der Kolonne (Kolonnensumpf) zugeführt wird.

4. Verfahren nach Anspruch 2, **dadurch gekennzeichnet, daß** man einen Teil des Stroms der aromatischen Verbindung wiedereinsetzt in derselben Einheit Mischer-Dekanter, wobei man den anderen Teil einer Einheit Mischer-Dekanter zuführt, die sich oberhalb oder unterhalb dieser Einheit befindet, je nachdem, ob die gewählte Betriebsweise eine Extraktion im Gegenstrom ist oder nicht.

5. Verfahren nach dem vorhergehenden Anspruch, **dadurch gekennzeichnet, daß** Verfahrensschritt (a) in mindestens zwei Einheiten Mischer-Dekanter, die im Gegenstrom arbeiten, durchgeführt wird.

6. Verfahren nach einem der vorhergehenden Ansprüche, **dadurch gekennzeichnet, daß** das Inkontaktbringen in Verfahrensschritt (a) bei einer Temperatur zwischen 10 und 70 °C, vorzugsweise zwischen 40 und 70 °C, erfolgt.

7. Verfahren nach einem der vorhergehenden Ansprüche, **dadurch gekennzeichnet, daß** man das Inkontaktbringen in Verfahrensschritt (a) mit einem Gewichtsverhältnis von wäßriger Lösung zu aromatischer Verbindung zwischen 1/1 und 10/1 durchführt.

8. Verfahren nach einem der vorhergehenden Ansprüche, **dadurch gekennzeichnet, daß** man die Destillation in Verfahrensschritt (c) in mindestens einer Kolonne durchführt.

9. Verfahren nach einem der vorhergehenden Ansprüche, **dadurch gekennzeichnet, daß** man eine Destillation durchführt, um eine Lösung von Säuren zu erhalten, deren Gesamtkonzentration an Säuren zwischen 30 und 75 % liegt.

10. Verfahren nach einem der vorhergehenden Ansprüche, **dadurch gekennzeichnet, daß** man die Destillation in Verfahrensschritt (c) in zwei Kolonnen durchführt:
• die erste Kolonne, in der ein Teil des Wassers verdampft wird,
• die zweite Kolonne, in der man die Verdampfung durchführt, bis man die gewünschte Konzentration an Säuren erhält, wobei dieser Kolonne die am Fuß der vorhergehenden Kolonne erhaltene flüssige Fraktion zugeführt wird und diese Kolonne durch den thermischen Fluß aufgeheizt wird, der bei der Kondensation des Wassers am Kopf der ersten Kolonne erhalten wird.

11. Verfahren nach einem der vorhergehenden Ansprüche, **dadurch gekennzeichnet, daß** man die wäßrigen Lösungen, die aus der Wäsche von aromatischen Verbindungen stammen, die ausgewählt sind aus Mononitrobenzol, Dinitrobenzol und seinen Isomeren sowie Mononitrotoluol, Dinitrotoluol und seinen Isomeren, behandelt.

12. Verfahren nach einem der vorhergehenden Ansprüche, **dadurch gekennzeichnet, daß** man Verfahrensschritt (a) mit einer aromatischen Verbindung durchführt, die ausgewählt ist aus Benzol und Toluol.

## Claims

1. A process for treating an aqueous solution from washing mononitro or dinitro compounds obtained by reacting a corresponding aromatic compound with a' nitrating acid comprising at least nitric acid, said aromatic mononitro or dinitro compounds having been separated from the aqueous acidic phase prior to said washing; said washing being carried out with water that may also comprise traces of nitrating acid, **characterized in that** the following steps are carried out:
a) said aqueous solution is brought into contact with said aromatic compound to obtain an aqueous phase and an organic phase;
b) said organic phase is recycled to the nitration process or to the step for extracting residual mononitration acids;
c) said aqueous phase is distilled;
d) the concentrated acidic solution obtained is recycled to the nitration process;
e) the water recovered after distillation is recycled or eliminated.

2. A process according to the preceding claim, **characterized in that** step a) is carried out in an extraction column or in at least one mixer-settler unit, preferably at least two mixersettler units.

3. A process according to the preceding claim, **characterized in that** step a) is carried out in an extraction column wherein the aqueous solution is supplied to the head of the column and the aromatic compound is supplied to the bottom of the column.

4. A process according to claim 2, **characterized in that** a portion of the stream of aromatic compound is recycled to the same mixer-settler unit, the other portion being supplied to a mixer-settler unit located upstream or downstream, depending on whether the operating mode is respectively counter-current extraction or otherwise.

5. A process according to the preceding claim, **characterized in that** step a) is carried out in at least two mixer-settler units, operating in counter-current mode.

6. A process according to any one of the preceding claims, **characterized in that** contact during step a) is carried out at a temperature in the range 10°C to 70°C, preferably in the range 40°C to 70°C.

7. A process according to any one of the preceding claims, **characterized in that** contact during step a) is carried out with a ratio of the aqueous solution to the aromatic compound that is in the range 1/1 to 10/1 by weight.

8. A process according to any one of the preceding claims, **characterized in that** the distillation of step c) is carried out in at least one column.

9. A process according to any one of the preceding claims, **characterized in that** the distillation is carried out to obtain a solution of acids with a total acid concentration in the range 30% to 75%.

10. A process according to any one of the preceding claims, **characterized in that** the distillation of step c) is carried out in two columns:
• a first column in which a portion of the water is evaporated off;
• a second column, in which evaporation is carried out until the desired concentration of acids is produced, it being supplied with the liquid fraction recovered from the bottom of the preceding column, and heated by the heat recovered from condensing water at the head of the first column.

11. A process according to any one of the preceding claims, **characterized in that** aqueous solutions from washing aromatic compounds selected from mononitrobenzene, dinitrobenzene and its isomers, mononitrotoluene, dinitrotoluene and its isomers are treated.

12. A process according to any one of the preceding claims, **characterized in that** step a) is carried out with an aromatic compound selected from benzene and toluene.
